# EUROPEAN PATENT APPLICATION

(11) **EP 2 829 537 A1**
(43) Date of publication of application: **28.01.2015**
(21) Application number: 13764058.7
(22) Date of filing: 11.03.2013
(51) Int. Cl.: C07D 311/78, C07C 323/21, C07D 265/30, C07D 277/60, C07D 279/14, C07D 327/04, C07D 497/04, C07D 513/04, C09K 9/02

(54) **CHROMENE COMPOUND**

(30) Priority: 21.03.2012 JP 2012063353
(71) Applicant: Tokuyama Corporation, Shunan-shi, Yamaguchi 745-0053 (JP)
(72) Inventor: IZUMI, Shinobu, Shunan-shi Yamaguchi 745-0053 (JP); SHIMIZU, Yasutomo, Shunan-shi Yamaguchi 745-0053 (JP); MOMODA, Junji, Shunan-shi Yamaguchi 745-0053 (JP)
(74) Representative: Adam, Holger
(86) International application number: PCT/JP2013/057330
(87) International publication number: WO 2013/141143

(57) **Abstract**

A novel photochromic compound which develops a color of a neutral tint (double peak characteristic) and has high color optical density, high fading speed and excellent durability.

Like a compound represented by the following formula (20), the photochromic compound having a group (methylthio group in the following formula 20) which has a sulfur atom bonded to the 7-position carbon atom is a photochromic compound having high color optical density, high fading speed and excellent durability.

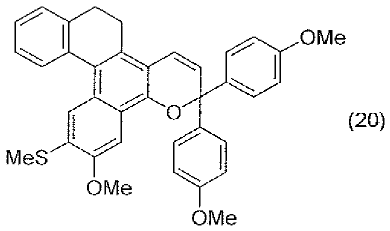

## Description

### TECHNICAL FIELD

The present invention relates to a novel chromene compound which is useful as a photochromic compound for photochromic spectacle lenses.

### BACKGROUND ART

Photochromism is the reversible function of a certain compound that it changes its color swiftly upon exposure to light including ultraviolet light such as sunlight or light from a mercury lamp and returns to its original color when it is put in the dark by stopping its exposure to light. A compound having this property is called "photochromic compound" and used as a material for photochromic plastic lenses.

For the photochromic compound used for this purpose, the following properties are required: (I) the degree of coloration at a visible light range before ultraviolet light is applied (to be referred to as "initial coloration" hereinafter) should be low, (II) the degree of coloration upon exposure to ultraviolet light (to be referred to as "color optical density" hereinafter) should be high, (III) the speed from the time when the application of ultraviolet light is started to the time when the color optical density reaches saturation (to be referred to as "color development sensitivity" hereinafter) should be high; (IV) the speed from the stoppage of the application of ultraviolet light to the time when the compound returns to its original state (to be referred to as "fading speed" hereinafter) should be high, (V) the repeat durability of this reversible function should be high, and (VI) the compound should dissolve in a monomer composition which will become a host material after curing to such a high concentration that its dispersibility in the host material in use becomes high.

As the photochromic compound which can satisfy these requirements, there are known chromene compounds having an indeno(2,1-f)naphtho(1,2-b)pyran structure as the basic skeleton and chromene compounds having a basic skeleton composed of six-membered condensation rings represented by the following formula (this basic skeleton may be referred to as "condensation six-membered ring skeleton" hereinafter).

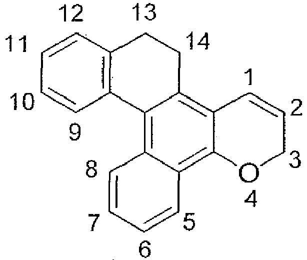

It is preferred that a photochromic plastic lens comprising a photochromic compound should develop a color of a neutral tint such as gray or brown. A color of a neutral tint is obtained by mixing together several different kinds of photochromic compounds which develop different colors. Stated more specifically, it can be obtained by mixing together a yellow to red photochromic compound (yellow compound) having a maximum absorption at 430 to 530 nm and a purple to blue photochromic compound (blue compound) having a maximum absorption at 550 to 650 nm.

However, when color control is carried out by this method, various problems occur due to the difference in photochromic properties between the compounds which have been mixed together. For example, when the repeat durability of the yellow compound is lower than that of the blue compound and the photochromic plastic lens is used for a long time, there occurs a problem that the developed color gradually changes to a color of a strong blue tint. Further, when the color development sensitivity and fading speed of the yellow compound are lower than those of the blue compound, there arises a problem that color during development has a strong blue tint and color during fading has a strong yellow tint.

It is considered that this problem can be solved by using a single compound which has two or more absorption maximums at the time of exposure and develops a color of a neutral tint (this compound may be simply referred to as "double peak compound" hereinafter). It is known that the yellow compound is generally inferior to the blue compound in durability. Therefore, a compound having higher yellow color optical density (maximum absorption wavelength of 430 to 530 nm) than blue color optical density (maximum absorption wavelength of 550 to 650 nm) is desired as the double peak compound (the ratio of the yellow color optical density to the blue color optical density in the double peak compound may be referred to as "double peak characteristic" hereinafter). Out of the double peak compounds, a chromene compound having the above condensation six-membered ring skeleton as the basic skeleton has high double peak characteristic. As the double peak compound having two or more absorption maximums at the time of color development and a condensation six-membered ring skeleton, there are known compounds represented by the following formulas (A) to (C).

However, these compounds have room for the improvement of the following points. That is, a chromene compound represented by the following formula (A) (refer to a pamphlet of International Laid-Open WO00/15628) has low fading speed and low repeat durability though its double peak characteristic is high.

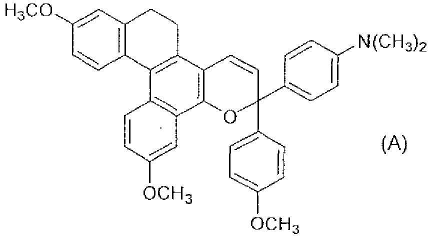

A chromene compound represented by the following formula (B) (refer to a pamphlet of International Laid-Open WO02/090342) has room for the improvement of initial coloration and repeat durability though it has high fading speed.

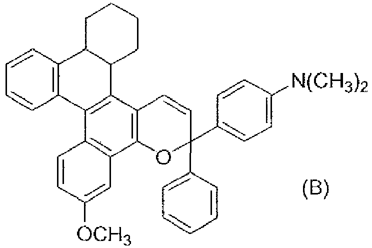

A chromene compound represented by the following formula (C) (refer to a pamphlet of International Laid-open WO07/086532) has slightly low double peak characteristic and room for the improvement of fading speed though its repeat durability is improved.

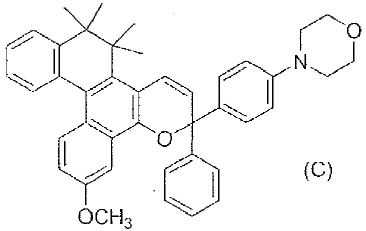

### DISCLOSURE OF THE INVENTION

It is therefore an object of the present invention to provide a chromene compound which develops a color of a neutral tint and is improved in photochromic properties as compared with the above compounds.

It is another object of the present invention to provide a chromene compound which has little initial coloration and high fading speed and rarely experiences the reduction of color optical density when it is used repeatedly, that is, excellent in the durability of photochromic properties.

It is still another object of the present invention to provide a novel naphthol compound for the manufacture of the chromene compound of the present invention.

Other objects and advantages of the present invention will become apparent from the following description.

The double peak compound having a condensation six-membered ring skeleton can exhibit excellent double peak characteristic. The inventors of the present invention tried to introduce various substituents into this chromene compound having a condensation six-membered ring skeleton. As a result, they found that a chromene compound having high color optical density, high fading speed and little initial coloration by thermochromism while retaining high double peak characteristic is obtained by introducing a group having a sulfur atom which is bonded to either one of carbon atoms at specific positions, specifically the 6-position and the 7-position of the above condensation six-membered ring skeleton. The present invention was accomplished based on this finding.

According to the present invention, the above objects and advantages of the present invention are attained by a chromene compound represented by the following formula (1). 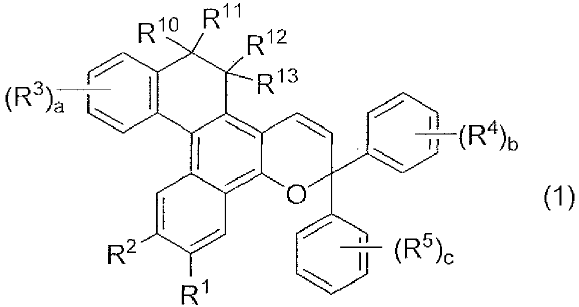 {In the above formula, R¹ and R² are defined by the following (i) or (ii):
(i) Either one or both of R¹ and R² are sulfur-containing groups represented by the following formula (2), or either one of them is a sulfur-containing group represented by the formula (2) and the other is a hydrogen atom, hydroxyl group, alkyl group, haloalkyl group, cycloalkyl group, alkoxy group, amino group, heterocyclic group bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group.

   -S-R⁶ (2)

   (wherein R⁶ is a hydrogen atom, alkyl group, cycloalkyl group or aryl group.)
(ii) R¹ and R² are bonded together to form a group represented by the following formula (3): 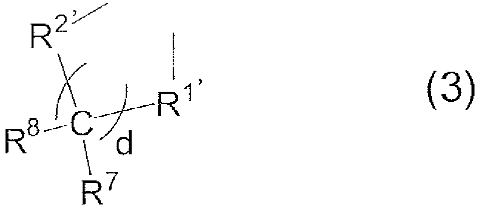 (wherein either one or both of R¹' and R²' are sulfur-atoms, or either one of them is a sulfur atom and the other is a methylene group, oxygen atom or group represented by the following formula (4): 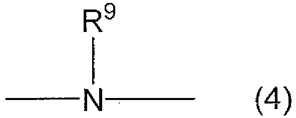 (wherein R⁹ is a hydrogen atom, hydroxyl group, alkyl group, haloalkyl group, cycloalkyl group, alkoxy group, halogen atom, aralkyl group, aralkoxy group, aryloxy group or aryl group.)
   R⁷ and R⁸ are each a hydrogen atom, hydroxyl group, alkyl group, haloalkyl group, cycloalkyl group, alkoxy group, amino group, heterocyclic group bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group, R⁷ and R⁸ may be bonded together to form an aliphatic ring having 3 to 20 ring member carbon atoms together with carbon atoms bonded thereto, "d" is an integer of 1 to 3.)
   R³, R⁴ and R⁵ are each a hydroxyl group, alkyl group, haloalkyl group, cycloalkyl group, alkoxy group, amino group, heterocyclic group bonded to a benzene ring bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group, aryl group, alkylthio group, cycloalkylthio group or arylthio group, "a" is an integer of 0 to 4, "b" and "c" are each an integer of 0 to 5, when "a" is 2 to 4, a plurality of R³' s may be the same or different, and when "b" and "c" are each 2 to 5, a plurality of R⁴' s and a plurality of R⁵' s may be the same or different, R¹⁰, R¹¹, R¹² and R¹³ are each a hydrogen atom, alkyl group, cycloalkyl group or aryl group, and two groups selected from R¹⁰, R¹¹, R¹² and R¹³ may be bonded together to form a ring together with carbon atoms bonded thereto.}

According to the present invention, secondly, the above objects and advantages of the present invention are attained by a photochromic curable composition which comprises the above chromene compound of the present invention and polymerizable monomers.

According to the present invention, thirdly, the above objects and advantages of the present invention are attained by a photochromic optical article having a polymer molded body containing the above chromene compound of the present invention dispersed therein as a constituent member. According to the present invention, in the fourth place, the above objects and advantages of the present invention are attained by an optical article having an optical substrate at least one surface or all of which is covered with a polymer film containing the above chromene compound of the present invention dispersed therein as a constituent member.

Further, according to the present invention, in the fifth place, the above objects and advantages of the present invention are attained by a naphthol compound represented by the following formula (5).

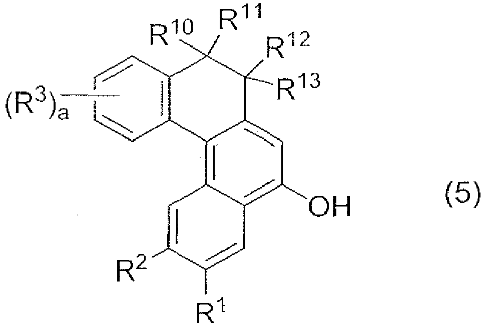

In the above formula, R¹, R², R³, R¹⁰, R¹¹, R¹², R¹³ and "a" are as defined in the above formula (1).

### BEST MODE FOR CARRYING OUT THE INVENTION

The chromene compound of the present invention is represented by the following formula (1).

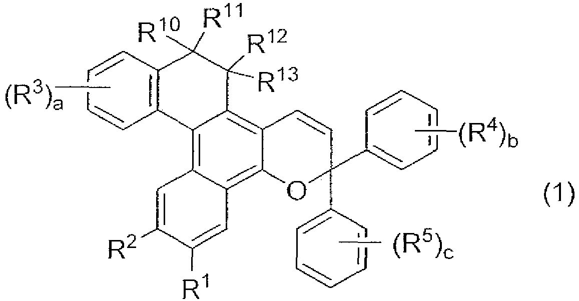

This compound having the above condensation six-membered ring skeleton, wherein at least one of R¹ and R² has a sulfur atom bonded to a carbon atom at the 6-position or the 7-position of the condensation six-membered ring skeleton, has been unknown up till now. A description is subsequently given of the above compound.

### <R¹ and R²>

R¹ and R² are defined by the following (i) or (ii). (i) Either one or both of R¹ and R² have a sulfur atom which is bonded to a carbon atom at the 6-position or the 7-position of a condensation six-membered ring skeleton. In this case, R¹ and R² are independent groups. In the case (ii), R¹ and R² form a ring together with carbon atoms at the 6-position and the 7-position of the condensation six-membered ring skeleton.

### A description is first given of the case (i)

Either one or both of R¹ and R² are sulfur-containing groups represented by the following formula (2).

-S-R⁶ (2)

As a matter of course, S denotes a sulfur atom in the above formula (2).

R⁶ is a hydrogen atom, alkyl group, cycloalkyl group or aryl group.

The above alkyl group is preferably an alkyl group having 1 to 6 carbon atoms. Preferred examples of the alkyl group include methyl group, ethyl group, n-propyl group, isopropyl group, n-butyl group, sec-butyl group, tert-butyl group, pentyl group and hexyl group.

The above cycloalkyl group is preferably a cycloalkyl group having 3 to 8 carbon atoms. Preferred examples of the cycloalkyl group include cyclopropyl group, cyclobutyl group, cyclopentyl group and cyclohexyl group.

The above aryl group is preferably an aryl group having 6 to 14 carbon atoms. Preferred examples of the aryl group include phenyl group, 1-naphtyl group and 2-naphthyl group. 1 to 7 hydrogen atoms, particularly preferably 1 to 4 hydrogen atoms of the benzene or naphthalene ring of the aryl group may be substituted by the above alkyl group having 1 to 6 carbon atoms, cycloalkyl group having 3 to 8 carbon atoms, alkoxy group having 1 to 6 carbon atoms or hydrogen atom. Examples of such substituted aryl group include toluyl group, xylyl group, cyclohexylphenyl group, chlorophenyl group, dichlorophenyl group, methoxyphenyl group and methoxyxylyl group.

Out of these, R⁶ is preferably an alkyl group or aryl group since high double peak characteristic is obtained and raw materials are easily acquired and particularly preferably an aryl group having a substituent since heat resistance becomes high.

Preferred examples of R⁶ include methyl group, ethyl group, phenyl group, 2-methylphenyl group and 2,6-dimethylphenyl group.

Both of R¹ and R² may be sulfur-containing groups represented by the above formula (2), or either one of them may be a sulfur-containing group represented by the above formula (2). When either one of them is a sulfur-containing group, the other is a hydrogen atom, hydroxyl group, alkyl group, haloalkyl group, cycloalkyl group, alkoxy group, amino group, heterocyclic group bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group. A description is subsequently given of these other groups.

Out of the other groups, the alkyl group, the cycloalkyl group and the aryl group are the same as those explained for R⁶.

The above haloalkyl group is preferably an alkyl group having 1 to 6 carbon atoms and substituted by a fluorine atom, chlorine atom or bromine atom. Preferred examples of the haloalkyl group include trifluoromethyl group, tetrafluoroethyl group, chloromethyl group, 2-chloroethyl group and bromomethyl group.

The above alkoxy group is preferably an alkoxy group having 1 to 6 carbon atoms. Preferred examples of the alkoxy group include methoxy group, ethoxy group, n-propoxy group, isopropoxy group, n-butoxy group, sec-butoxy group and tert-butoxy group.

The above amino group is not limited to an amino group (-NH²) and may be substituted by one or two hydrogen atoms. Examples of the substituent of the amino group include alkyl groups having 1 to 6 carbon atoms, haloalkyl groups having 1 to 6 carbon atoms, alkoxy group shaving 1 to 6 carbon atoms, cycloalkyl groups having 3 to 7 carbon atoms, aryl groups having 6 to 14 carbon atoms and heteroaryl groups having 4 to 14 carbon atoms. Preferred examples of the amino group include amino group, methylamino group, dimethylamino group, ethylamino group, diethylamino group, phenylamino group and diphenylamino group.

The above heterocyclic group bonded to a carbon atom bonded thereto via a ring member nitrogen atom is a 5- to 7-membered aliphatic or aromatic heterocyclic group containing 1 to 2 nitrogen atoms and optionally one oxygen atom or sulfur atom as ring constituent atoms, or a condensation heterocyclic group having a cyclohexane ring or benzene ring condensed to these groups. Examples of these heterocyclic groups include aliphatic heterocyclic groups such as morpholino group, piperidino group, pyrrolidinyl group, piperazino group and N-methylpiperazino group, and aromatic heterocyclic groups such as indolinyl group. A preferred example of the substituent of the heterocyclic group is an alkyl group. Preferred examples of the heterocyclic group having a substituent include 2,6-dimethylmorpholino group, 2,6-dimethylpiperidino group and 2,2,6,6-tetramethylpiperidino group.

The above alkylcarbonyl group is preferably an alkylcarbonyl group having 2 to 7 carbon atoms. Preferred examples of the alkylcarbonyl group include acetyl group and ethylcarbonyl group.

The above alkoxycarbonyl group is preferably an alkoxycarbonyl group having 2 to 7 carbon atoms. Preferred examples of the alkoxycarbonyl group include methoxycarbonyl group and ethoxycarbonyl group.

Examples of the above halogen atom include fluorine atom, chlorine atom, bromine atom and iodine atom.

The above aralkyl group is preferably an aralkyl group having 7 to 11 carbon atoms. Preferred examples of the aralkyl group include benzyl group, phenylethyl group, phenylpropyl group, phenylbutyl group and naphthylmethyl group.

The above aralkoxy group is preferably an aralkoxy group having 7 to 11 carbon atoms. Preferred examples of the aralkoxy group include benzyloxy group and naphthylmethoxy group.

The above aryloxy group is preferably an aryloxy group having 6 to 12 carbon atoms. Preferred examples of the aryloxy group include phenyloxy group and naphthyloxy group.

1 to 7 hydrogen atoms, particularly preferably 1 to 4 hydrogen atoms of the benzene or naphthalene ring of each of the aralkyl group, the aralkoxy group and the aryloxy group may be substituted by the above hydroxyl group, alkyl group, haloalkyl group, cycloalkyl group, alkoxy group, amino group, heterocyclic group, cyano group, nitro group, formyl group, hydroxylcarbonyl group, alkylcarbonyl group, alkoxycarbonyl group or halogen atom.

### In re features of combinations in case (i)

When both of R¹ and R² are sulfur-containing groups represented by the above formula (2), the obtained chromene compound has excellent photochromic properties and high double peak characteristic. When R¹ is a sulfur-containing group represented by the above formula (2) and R² is another group, the obtained chromene compound has high color optical density. Further, when R² is a sulfur-containing group represented by the above formula (2) and R¹ is another group, the obtained chromene compound has a short absorption end and little initial coloration.

### In case (ii)

R¹ and R² may be bonded together to form a group represented by the following formula (3).

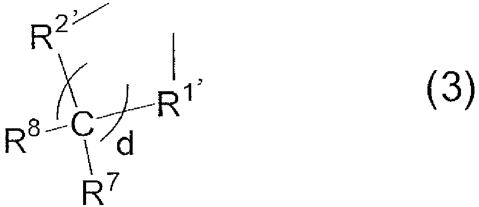

In the above formula, either one or both of R^{1'} and R^{2'} are sulfur atoms.

At least one of R^{1'} and R^{2'} may be a sulfur atom, or both of them may be sulfur atoms. When either one of them is a sulfur atom, the other is a methylene group, oxygen atom or group represented by the following formula (4).

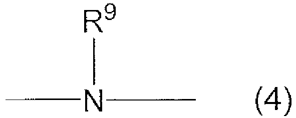

In the above formula (3), R⁷ and R⁸ are each a hydrogen atom, hydroxyl group, alkyl group, haloalkyl group, cycloalkyl group, alkoxy group, amino group, heterocyclic group bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group. These groups are the same as those explained for the above R⁶ and the other group in the case (i), and specific examples thereof are the same.

R⁷ and R⁸ may be bonded together to form an aliphatic ring having 3 to 20 ring member carbon atoms together with carbon atoms bonded thereto. Examples of the above aliphatic ring include cyclopentane ring, cyclohexane ring, cycloheptane ring and cyclooctane ring. The aliphatic ring is particularly preferably a cyclohexane ring out of these. 1 to 6 hydrogen atoms, particularly preferably 1 to 4 hydrogen atoms of each of these rings may be substituted by an alkyl group having 1 to 6 carbon atoms. The aliphatic ring is preferably substituted by an alkyl group since heat resistance becomes high, and preferred examples thereof include 2,2-dimethylcyclopentane ring,
2,2-dimethylcyclohexane ring and
2,2,6,6-tetramethylcyclohexane ring.

In the above formula (4), R⁹ is a hydrogen atom, hydroxyl group, alkyl group, haloalkyl group, cycloalkyl group, alkoxy group, halogen atom, aralkyl group, aralkoxy group, aryloxy group or aryl group. These groups are the same as those explained for the above R⁶ and the other group in the case (i), and specific examples thereof are the same.

Out of these, R⁹ is preferably a hydrogen atom or alkyl group since high double peak characteristic is obtained. R⁹ is particularly preferably a hydrogen atom, methyl group or ethyl group.

### <preferred R⁸ and R⁸>

In the present invention, preferred R⁷ and R⁸ are each a hydrogen atom, alkyl group, haloalkyl group, cycloalkyl group or aryl group, or R⁷ and R⁸ are bonded together to form a ring together with carbon atoms bonded thereto. R¹ and R⁸ are particularly preferably the same from the viewpoint of synthesis ease.

In order to enable the chromene compound of the present invention to have excellent photochromic properties, R⁷ and R⁸ are preferably groups other than a hydrogen atom. To enhance the heat resistance of an optical article containing the chromene compound of the present invention, R⁷ and R⁸ are preferably sterically bulky groups. A detailed description is subsequently given of the heat resistance of an optical article.

When an optical article containing an organic compound such as a chromene compound (for example, a photochromic plastic lens) is kept at a temperature of 100°C or higher for a long time, it gradually yellows or may change its developed color according to circumstances. This is considered to be because the organic compound contained in the optical article is degraded by oxidation. In a compound containing a sulfur atom in particular, it is considered that the sulfur atom is readily oxidized to form a sulfoxide (-SO-) or a sulfone (-SO₂-). Therefore, "heat resistance" as used herein can be reworded as "oxidation resistance". When the inventors of the present invention conducted intensive studies to improve this oxidation resistance, they found that when a sterically bulky substituent or a substituent which reduces electron density on a sulfur atom such as an aryl group is used as R⁶ and R⁷ in the present invention, the oxidation resistance of the optical article containing the chromene compound of the present invention is improved, thereby greatly improving stability at a high temperature. The sterically bulky group means bulkiness with respect to a sulfur atom. It is considered that as the substituent is more bulky, steric hindrance to the sulfur atom becomes higher with the result that an oxidation degradation reaction hardly occurs, thereby improving oxidation resistance.

Although the cause is not known, when a bulky substituent such as a secondary alkyl group or a tertiary alky group is used as R⁷ and R⁸, as shown in Examples, double peak characteristic is improved.

For the above reason, R⁷ and R⁸ are preferably sterically bulky groups in order to improve the heat resistance of an optical article containing the chromene compound of the present invention as described above. Stated more specifically, the alkyl group is preferably an alkyl group having 1 to 6 carbon atoms, more preferably a branched alkyl group having 3 to 6 carbon atoms. The alkyl group is particularly preferably an isopropyl group, isobutyl group or tert-butyl group. The cycloalkyl group is preferably a cycloalkyl group having 3 to 8 carbon atoms, particularly preferably cyclopentyl group or cyclohexyl group. The aryl group is preferably an aryl group having 6 to 20 carbon atoms. More specifically, the aryl group is particularly preferably a naphthyl group or phenyl group, and a 2-methylphenyl group having a methyl group as a substituent at the ortho-position of a phenyl group is also preferred. When R⁷ and R⁸ are bonded together to form a ring together with carbon atoms bonded thereto, the formed ring is preferably an aliphatic ring having 3 to 6 carbon atoms, specifically a cyclopentane ring or cyclohexane ring. The ring may have a methyl group as a substituent at carbon atoms adjacent to the carbon atoms bonded to R⁷ and R⁸, and examples thereof include
2,2-dimethylcyclohexane ring and
2,2,6,6-tetramethylcyclohexane ring.

### <d>

"d" is an integer of 1 to 3. When "d" is 2 or more, a plurality of groups represented by the following formula may be the same or different. 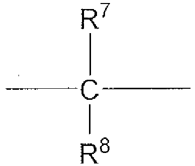 Out of these, "d" is preferably 1 or 2, particularly preferably 1 since high color optical density and high fading speed can be obtained at the same time.

### In re features of combination in case (ii)

When R^{1'} is a sulfur atom, the chromene compound of the present invention has little initial coloration. When R^{2'} is a sulfur atom, the chromene compound of the present invention has high durability. When either one of R^{1'} and R^{2'} is an oxygen atom, the chromene compound of the present invention has high fading speed.

Stated more specifically, when both R^{1'} and R^{2'} are sulfur atoms, the chromene compound of the present invention has excellent photochromic properties and high double peak characteristic. When R^{1'} is a sulfur atom and R^{2'} is a methylene group, oxygen atom or group represented by the formula (4), the chromene compound of the present invention has high color optical density. When R^{2'} is a sulfur atom and R^{1'} is a methylene group, oxygen atom or group represented by the formula (4), the chromene compound of the present invention has a short absorption end and little initial coloration.

### <R³, R⁴ and R⁵>

R³, R⁴ and R⁵ are each a hydroxyl group, alkyl group, haloalkyl group, cycloalkyl group, alkoxy group, amino group, heterocyclic group bonded to a benzene ring (carbon atom) bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group, aryl group, alkylthio group, cycloalkylthio group or arylthio group.

Out of the above groups, the alkyl group, the haloalkyl group, the cycloalkyl group, the alkoxy group, the amino group, the heterocyclic group bonded to a benzene ring (carbon atom) bonded thereto via a ring member nitrogen atom, the alkylcarbonyl group, the alkoxycarbonyl group, the halogen atom, aralkyl group, the aralkoxy group, the aryloxy group and the aryl group are the same as those explained for the above R⁶ and the other group in the case (i), and specific examples thereof are the same.

The above alkylthio group is preferably an alkylthio group having 1 to 6 carbon atoms. Preferred examples of the alkylthio group include methylthio group, ethylthio group, n-propylthio group, isopropylthio group, n-butylthio group, sec-butylthio group and t-butylthio group.

The above cycloalkylthio group is preferably a cycloalkylthio group having 3 to 8 carbon atoms. Preferred examples of the cycloalkylthio group include cyclopropylthio group, cyclobutylthio group, cyclopentylthio group and cyclohexylthio group.

The above arylthio group is preferably an arylthio group having 6 to 10 carbon atoms. Preferred examples of the arylthio group include phenylthio group, 1-naphthylthio group and 2-naphthylthio group.

1 to 9 hydrogen atoms, particularly preferably 1 to 4 hydrogen atoms of each of the arylthio group and the cycloarylthio group may be substituted by an alkyl group having 1 to 6 carbon atoms, alkoxy group having 1 to 6 carbon atoms, cycloalkyl group having 3 to 8 carbon atoms or halogen atom.

"a" is an integer of 0 to 4, indicative of the number of R^{3'} s. When "a" is 2, two R^{3'} s may be the same or different. "b" and "c" are each an integer of 0 to 5, indicative of the numbers of R^{4'}s and R^{5'}s, respectively.

Out of the above groups, R³ is preferably a hydrogen atom ("a" is 0), alkyl group or alkoxy group as high color optical density is obtained. R³ is particularly preferably a hydrogen atom, methyl group or methoxy group and bonded to the 11-position carbon atom.

At least one, preferably both of R⁴ and R⁵ are groups selected from alkyl group, alkoxy group, amino group and heterocyclic group bonded to a benzene ring bonded thereto via a ring member nitrogen atom since the obtained chromene compound exhibits excellent photochromic properties. To exhibit this effect fully, R⁴ and R⁵ are preferably bonded to the para-position with respect to the carbon atom of a benzene ring bonded to the 3-position carbon atom in the skeleton of the formula (1).

### <R¹⁰, R¹¹, R¹² and R¹³>

R¹⁰, R¹¹, R¹² and R¹³ are each a hydrogen atom, alkyl group, cycloalkyl group or aryl group.

Two groups selected from R¹⁰, R¹¹, R¹² and R¹³ may be bonded together to from a ring together with carbon atoms bonded thereto.

The alkyl group, the cycloalkyl group and the aryl group are the same as those explained for R⁶, and specific examples thereof are the same.

When two groups selected from R¹⁰, R¹¹, R¹² and R¹³ are bonded together to form a ring together with carbon atoms bonded thereto, the formed ring is an aliphatic ring having preferably 5 to 20 carbon atoms, particular preferably 5 to 8 carbon atoms. The ring may be substituted by 1 to 4 alkyl groups having 1 to 4 carbon atoms. Preferred examples of the aliphatic ring include cyclopentyl group, cyclohexyl group, cycloheptyl group, dimethycyclohexyl group and tetramethylcyclohexyl group.

### <preferred chromene compound>

Particularly preferred examples of the chromene compound of the present invention are compounds represented by the following formulas. In the formulas, Me and Pr denote methyl group and propyl group, respectively.

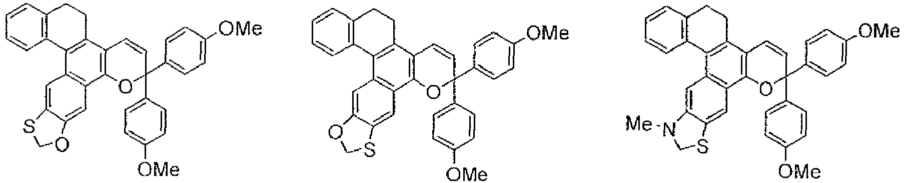

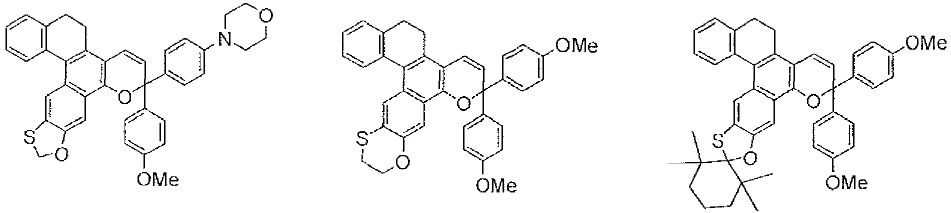

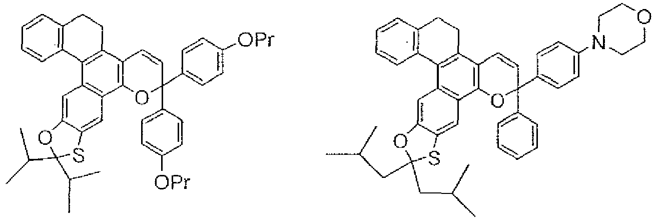

### (identification of chromene compound)

The chromene compound of the present invention is generally existent as an achromatic, light yellow or light green solid or viscous liquid at normal temperature and normal pressure and can be confirmed by the following means (1) to (3).
(1) When the proton nuclear magnetic resonance spectrum (¹H-NMR) of the chromene compound is measured, peaks based on an aromatic proton and an alkene proton appear at δ of around 5.0 to 9.0 ppm and peaks based on the protons of an alkyl group and an alkylene group appear at 5 of around 0.5 to 4.9 ppm. By comparing these spectral intensities relatively, the number of the protons of each of the bonds can be known.
(2) The composition of a corresponding product can be determined by elemental analysis.
(3) When the ¹³C-nuclear magnetic resonance spectrum (¹³C-NMR) of the chromene compound is measured, peaks based on the carbons of an aromatic hydrocarbon group appears at δ of around 110 to 160 ppm, peaks based on the carbons of an alkene appear at δ of around 80 to 140 ppm, and peaks based on the carbons of an alkyl group and an alkylene group appear at 5 of around 20 to 80 ppm.

### <production of chromene compound>

The process for producing the chromene compound of the present invention is not particularly limited and may be any synthetic process. For example, the chromene compound represented by the above formula (1) can be advantageously produced by the following process.

That is, the chromene compound can be advantageously produced by reacting a naphthol compound represented by the following formula (5) with a propargyl alcohol compound represented by the following formula (6) in the presence of an acid catalyst. 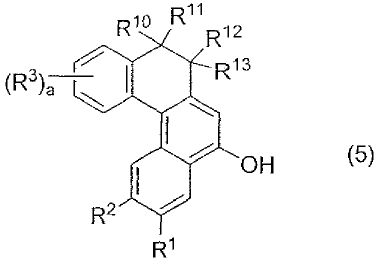 (wherein R¹, R², R³, R¹⁰, R¹¹, R¹², R¹³ and "a" are as defined in the above formula (1).) 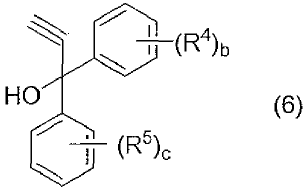 (wherein R⁴, R⁵, "b" and "c" are as defined in the above formula (1).)
The reaction ratio of the naphthol compound to the propargyl alcohol compound is selected from a wide range, preferably a range of 1:10 to 10:1 (molar ratio). Sulfuric acid, benzenesulfonic acid, p-toluenesulfonic acid or acid alumina is used as the acid catalyst in an amount of 0.1 to 10 parts by weight based on 100 parts by weight of the total of the naphthol compound and the propargyl alcohol compound. The reaction temperature is preferably 0 to 200°C. An aprotic organic solvent such as N-methylpyrrolidone, dimethyl formamide, tetrahydrofuran, benzene or toluene is preferably used as the solvent. The method of purifying the product obtained through the above reaction is not particularly limited. For example, the obtained product may be purified by carrying out silica gel column purification and further recrystallization.

### <method of synthesizing naphthol compound>

Although the method of synthesizing a naphthol compound represented by the above formula (5) is not particularly limited, the naphthol compound can be synthesized as follows, for example.

To begin with, a benzene compound represented by the following formula (7) may be purchased as a commercial product or may be synthesized based on the following document. 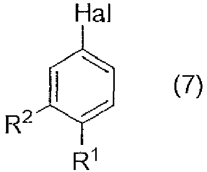 (In the above formula, Hal is a chlorine atom, bromine atom or iodine atom.)

In the above formula (7), R¹ and R² are as defined in the above formula (1).

For example, a benzene compound represented by the following formula (8) (Me denotes a methyl group, which shall be applied hereinafter) can be purchased as a reagent, and a benzene compound represented by the following formula (9) can be synthesized in accordance with a reaction method described in research papers such as Journal of Heterocyclic Chemistry, 19, 135 (1982).

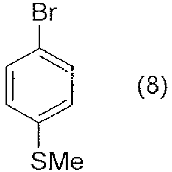

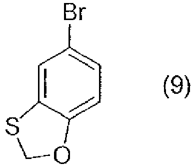

Thereafter, a Grignard reagent is prepared by reacting the benzene compound represented by the above formula (7) with metal magnesium and then reacted with a tetralone derivative represented by the following formula (10), and the reaction product is treated with an acid to obtain a compound represented by the following formula (11).

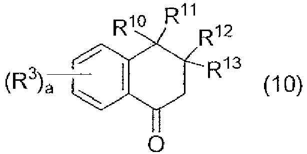

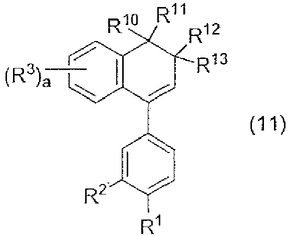

The compound of the formula (11) is brominated by using N-bromosuccinimide, reacted with a base and further dehydrated to obtain a compound represented by the following formula (12).

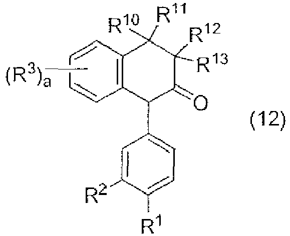

A cyanoacetic acid ester compound is added to this compound, and the resulting adduct is hydrolyzed by using a base to obtain a carboxylic acid compound represented by the following formula (13).

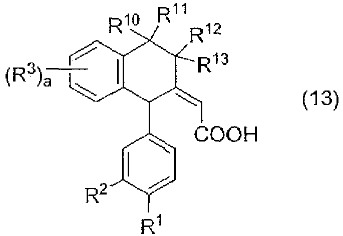

This compound is cyclized by using acetic anhydride and then subjected to a hydrolysis reaction so as to synthesize a naphthol compound of interest represented by the above formula (5).

### (propargyl alcohol compound)

The propargyl alcohol compound represented by the above formula (6) can be synthesized by various methods. For example, it can be easily synthesized by reacting a ketone compound with a metal acetylene compound such as lithium acetylide.

The chromene compound of the present invention is obtained by reacting the above naphthol compound with the propargyl alcohol compound. The obtained chromene compound dissolves well in a general-purpose organic solvent such as toluene, chloroform or tetrahydrofuran. When the chromene compound represented by the above formula (1) is dissolved in such a solvent, the obtained solution is almost achromatic and transparent and exhibits an excellent photochromic function that it develops a color swiftly upon exposure to sunlight or ultraviolet radiation and reversibly returns to its original achromatic state swiftly by blocking the light.

### (combination with another photochromic compound>

Although the chromene compound of the present invention develops a color of a neutral tint by itself, it may be used in combination with another photochromic compound to obtain various colors required as a photochromic lens. The chromene compound of the present invention produces an excellent effect. Therefore, even when the chromene compound is mixed with another photochromic compound to carry out color control, the obtained photochromic composition produces an excellent effect. Consequently, any known photochromic compound may be used as the photochromic compound to be combined with. Examples of the photochromic compound include fulgide, fulgimide, spirooxazine and chromene. Out of these, a chromene compound is particularly preferred because it can keep a color uniformly at the time of color development and fading, can suppress color drift at the time of color development due to the deterioration of photochromic properties and further can reduce initial coloration.

To obtain a photochromic composition comprising the chromene compound of the present invention and another chromene compound, the ratio of these chromene compounds may be suitably determined according to a desired color. To obtain a photochromic curable composition comprising this photochromic composition and polymerizable monomers, the total amount of the chromene compound of the present invention and another chromene compound is preferably 0.001 to 10 parts by mass based on 100 parts by mass of the total of all the polymerizable monomers. Stated more specifically, in the case of a thin film such as a coating film, for example, a thin film having a thickness of about 100 µm, color control should be carried out by using 0.001 to 5.0 parts by mass of the chromene compound of the present invention and 0.001 to 5.0 parts by mass of another chromene compound based on 100 parts by mass of the coating film or the total of all the polymerizable monomers which provide the coating film. In the case of a thick cured body, for example, a cured body having a thickness of 1 mm or more, color control should be carried out by using 0.001 to 0.5 part by mass of the chromene compound of the present invention and 0.001 to 0.5 part by mass of another chromene compound based on 100 parts by mass of the thick cured body or the total of all the polymerizable monomers which provide the thick cured body.

### (stabilizer to be combined with)

Although the chromene compound of the present invention has high durability as it is, its durability can be further enhanced by using the following ultraviolet absorbent, optical stabilizer or antioxidant. As the ultraviolet absorbent may be used known ultraviolet absorbents such as benzophenone-based compounds, benzotriazole-based compounds, cyanoacrylate-based compounds, triazine-based compounds and benzoate-based compounds. Cyanoacrylate-based compounds and benzophenone-based compounds are particularly preferred. When the above ultraviolet absorbent is added to a photochromic curable composition, it is used in an amount of 0.001 to 5 parts by mass based on 100 parts by mass of the total of all the polymerizable monomers so as to produce an effect. Known hindered amines may be used as the optical stabilizer, and known hindered phenols may be used as the antioxidant. When the above optical stabilizer or antioxidant is added to the photochromic curable composition, it is used in an amount of 0.01 to 10 parts by mass based on 100 parts by mass of the total of all the polymerizable monomers so as to produce an effect.

### (use of chromene compound)

The chromene compound of the present invention exhibits the same photochromic properties even in a polymer solid matrix. The polymer solid matrix is not particularly limited if the chromene compound of the present invention can be uniformly dispersed therein, and examples of the optically preferred polymer matrix include thermoplastic resins such as methyl polyacrylate, ethyl polyacrylate, methyl polymethacrylate, ethyl polymethacrylate, polystyrene, polyacrylonitrile, polyvinyl alcohol, polyacrylamide, poly(2-hydroxyethylmethacrylate), polydimethylsiloxane and polycarbonate.

A thermosetting resin obtained by polymerizing a radically polymerizable polyfunctional monomer may also be used as the above polymer matrix. Examples of the radically polymerizable polyfunctional monomer include polyacrylate and polymethacrylate compounds such as ethylene glycol diacrylate, diethylene glycol dimethacrylate, triethylene glycol dimethacrylate, tetraethylene glycol dimethacrylate, ethylene glycol bisglycidyl methacrylate, bisphenol A dimethacrylate, 2,2-bis(4-methacryloyloxyethoxyphenyl)propane and 2,2-bis(3,5-dibromo-4-methacryloyloxyethoxyphenyl) propane; polyallyl compounds such as diallyl phthalate, diallyl terephthalate, diallyl isophthalate, diallyl tartarate, diallyl epoxysuccinate, diallyl fumarate, diallyl chlorendate, diallyl hexaphthalate, diallyl carbonate, allyl diglycol carbonate and trimethylolpropane triallyl carbonate; polythioacrylate and polythiomethacrylate compounds such as 1,2-bis(methacryloylthio)ethane, bis(2-acryloylthioethyl)ether and 1,4-bis(methacryloylthiomethyl)benzene; acrylate and methacrylate compounds such as glycidyl acrylate, glycidyl methacrylate, β-methylglycidyl methacrylate, bisphenol A-monoglycidyl ether-methacrylate, 4-glycidyloxy methacrylate, 3-(glycidyl-2-oxyethoxy)-2-hydroxypropyl methacrylate, 3-(glycidyloxy-1-isopropyloxy)-2-hydroxypropyl acrylate and 3-glycidyloxy-2-hydroxypropyloxy)-2-hydroxypropyl acrylate; and divinyl benzene.

Copolymers obtained by copolymerizing the above radically polymerizable polyfunctional monomer with a radically polymerizable monofunctional monomer may also be used as the above polymer matrix. Examples of the radically polymerizable monofunctional monomer include unsaturated carboxylic acids such as acrylic acid, methacrylic acid and maleic anhydride; acrylate and methacrylate compounds such as methyl acrylate, methyl methacrylate, benzyl methacrylate, phenyl methacrylate and 2-hydroxyethyl methacrylate; fumarate compounds such as diethyl fumarate and diphenyl fumarate; thioacrylate and thiomethacrylate compounds such as methyl thioacrylate, benzyl thioacrylate and benzyl thiomethacrylate; and vinyl compounds such as styrene, chlorostyrene, methyl styrene, vinyl naphthalene, α-methylstyrene dimer and bromostyrene.

As the method of dispersing the chromene compound of the present invention into the above polymer solid matrix, commonly used methods may be employed. The methods include, for example, one in which the above thermoplastic resin and the chromene compound are kneaded together while they are molten to disperse the chromene compound into the resin, one in which the chromene compound is dissolved in the above polymerizable monomers and then a polymerization catalyst is added to polymerize the polymerizable monomers by heat or light so as to disperse the chromene compound into the resin, and one in which the surfaces of the above thermoplastic resin and the above thermosetting resin are dyed with the chromene compound to disperse the chromene compound into the resins.

The chromene compound of the present invention can be widely used as a photochromic material for use in, for example, recording materials as substitutes for silver halide photosensitive materials, copy materials, printing photosensitive materials, recording materials for cathode ray tubes, photosensitive materials for lasers and photosensitive materials for holography. A photochromic material comprising the chromene compound of the present invention may also be used as a photochromic lens material, optical filter material, display material or material for actinometers and ornaments.

For instance, when the chromene compound of the present invention is used in a photochromic lens, its production process is not particularly limited as long as uniform light control performance is obtained. An example of the process is such that a polymer film containing the photochromic material of the present invention uniformly dispersed therein is sandwiched between lenses. Another example is such that the chromene compound of the present invention is dispersed into the above polymerizable monomers and the polymerizable monomers are polymerized by a predetermined technique. A further example is such that this compound is dissolved in, for example, silicone oil, the resulting solution is impregnated into the surface of a lens at 150 to 200°C over 10 to 60 minutes, and the surface is further coated with a curable substance to obtain a photochromic lens. A still further example is such that the above polymer film is formed on the surface of a lens and the surface is coated with a curable substance to obtain a photochromic lens.

Moreover, a photochromic lens can also be manufactured by applying a coating agent composed of a photochromic curable composition comprising the chromene compound of the present invention to the surface of a lens substrate and curing the coating film. At this point, the lens substrate may be subjected to a surface treatment with an alkaline solution or a plasma treatment in advance, and a primer may be further applied so as to improve adhesion between the substrate and the coating film by carrying out or not carrying out the above surface treatment.

### EXAMPLES

The following examples are provided for the purpose of further illustrating the present invention but are in no way to be taken as limiting.

### Example 1

The following benzene compound (14) was first synthesized in accordance with a method described in Tetrahedron Letters, 39, 4657 (1998). 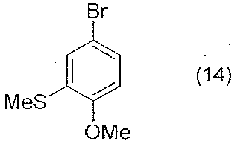 13.6 g (561 mmol) of magnesium was added to 1,120 ml of tetrahydrofuran and heated at 50°C, and 130.8 g (561 mmol) of the benzene compound of the above formula (14) was added dropwise to the resulting solution over 2 hours. Thereafter, a reaction was carried out at 65°C for 1 hour to prepare a Grignard solution.

1,120 ml of toluene was added to this solution, and 73.8 g (505 mmol) of 1-tetralone was added dropwise to the resulting solution so as to carry out a reaction at 50°C for 3 hours. After the end of the reaction, 299 g of 10 % hydrochloric acid was added to carry out a reaction at 30°C for 3 hours, the reaction solution was washed in water, the solvent was removed, and 477 ml of methanol was added for recrystallization to obtain a compound represented by the following formula (15) as 107.6 g (379 mmol, yield of 75%) of a white solid.

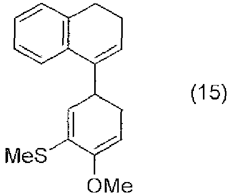

The compound of the above formula (15) was dissolved in a mixed solvent of 2, 150 ml of tetrahydrofuran and 1,076 ml of water and cooled to 0°C, and 74.6 g (417 mmol) of N-bromosuccinimide was added to the resulting solution to carry out a reaction at 20°C for 2 hours. The reaction solution was washed in water and brine. This reaction solution was cooled to 0°C, 46.8 g (417 mmol) of t-butoxypotassium was added to carry out a reaction at 20°C for 3 hours, the reaction solution was washed in water and brine, and the solvent was removed. 1, 900 ml of toluene and 10.1 g (94.7 mmol) of lithium perchlorate were added to the obtained product so as to carry out a reaction at 80°C for 3 hours. The reaction solution was washed in water, the solvent was removed, and 380 ml of 2-propanol was added for recrystallization so as to obtain a compound represented by the following formula (16) as 56.9 g (190 mmol, yield of 50 %) of a light yellow solid.

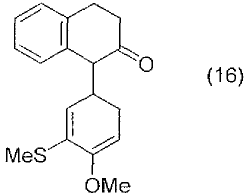

The compound of the above formula (16) was dissolved in 569 ml of toluene, and 23.6 g (208 mmol) of ethyl cyanoacetate, 8.8 g (114 mmol) of ammonium acetate and 28.4 g (474 mmol) of acetic acid were added to the resulting solution so as to carry out a reaction at 110°C for 3 hours. The reaction solution was washed in water, the solvent was removed, 1, 640 ml of ethanol was added, and 1, 950 g of a 5 % potassium hydroxide aqueous solution was added to carry out a reaction at 85°C for 18 hours. 1,650 ml of ethyl acetate was added to this reaction solution to extract a water layer. 1,650 ml of toluene was added to the obtained product, 605 g of a 36 % hydrochloric acid aqueous solution was added, and 850 ml of tetrahydrofuran was added to extract an organic layer. The solvent was concentrated, 320 ml of toluene was added, and the precipitated solid was filtered to obtain a carboxylic acid compound represented by the following formula (17) as 34.1 g (100 mmol, yield of 53 %) of a brown solid.

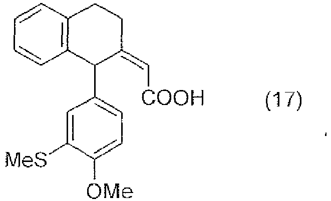

51.3 g (502 mmol) of acetic anhydride, 103 ml of toluene and 8.2 g (100 mmol) of sodium acetate were added to the compound of the above formula (17) to carry out a reaction at 110°C for 3 hours. The reaction solution was washed in water, the solvent was concentrated, 540 ml of methanol was added, and 0.3 g of a 36 % hydrochloric acid aqueous solution was added to carry out a reaction at 70°C for 2 hours. 2,714 ml of ethyl acetate was added to the reaction solution, the resulting solution was washed in water, the solvent was concentrated, and the obtained product was purified by column chromatography using silica gel to obtain a naphthol compound represented by the following formula (18) as 27.5 g (85.4 mmol, yield of 85 %) of a white solid.

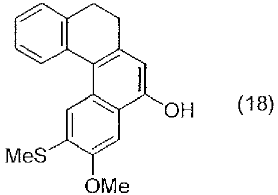

The elemental analysis values of this product were 74.68 % of C, 5.68 % of H and 9.87 % of S which were almost equal to the calculated values of C₂₀H₁₈O₂S (C: 74.50 %, H: 5.63 %, S: 9.95 %).

When the proton nuclear magnetic resonance spectrum was measured, it showed 10H peaks based on a methyl proton and a methylene proton at δ of around 1.0 to 5.0 ppm and 8H peaks based on an aromatic proton and the proton of a hydroxyl group at 5 of around 5.0 to 9.0 ppm. It was confirmed from the above results that the isolated product was a compound represented by the above formula (18).

3.22 g (10.0 mmol) of the above naphthol compound (18) and 3.30 g (12.5 mmol) of a propargyl alcohol compound represented by the following formula (19) were dissolved in 200 ml of toluene, and 0.022 g of p-toluenesulfonic acid was added and stirred under heating and reflux for 1 hour. 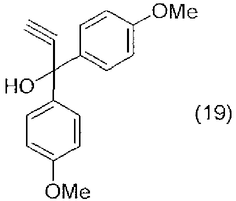 After a reaction, the solvent was removed, and the obtained product was purified by chromatography on silica gel to obtain 1.2 g of a white powdery product. The yield was 75 %.

The elemental analysis values of this product were 77.47 % of C, 5.77 % of H and 5.48 % of S which were almost equal to the calculated values of C₃₇H₃₂O₄S (C: 77.59 %, H: 5.63 %, S: 5.60 %).

When the proton nuclear magnetic resonance spectrum was measured, it showed a 4H peak based on a methylene proton at δ of around 1.0 to 3.0 ppm, 6H peaks based on the methyl protons of a methylthio group and a methoxy group at δ of around 2.3 to 6.0 ppm and 16H peaks based on an aromatic proton and an alkene proton at δ of around 5.6 to 9.0 ppm.

Further, when the ¹³C-nuclear magnetic resonance spectrum was measured, it showed peaks based on the carbons of an aromatic ring at δ of around 110 to 160 ppm, peaks based on the carbons of an alkene at δ of around 80 to 140 ppm and peaks based on the carbons of an alkyl at δ of around 20 to 60 ppm.

It was confirmed from the above results that the isolated product was a compound represented by the following formula (20).

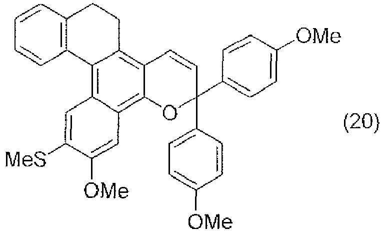

### Examples 2 to 16

Naphthol compounds were synthesized in the same manner as in Example 1 to synthesize chromene compounds shown in Table 1 (Examples 2 to 4), Table 2 (Examples 5 to 7), Table 3 (Examples 8 to 10), Table 4 (Examples 11 to 13) and Table 5 (Examples 14 to 16). When the structures of the obtained products were analyzed by means of the same structure confirming means as in Example 1, it was confirmed that they were compounds represented by structural formulas shown in Tables 1 to 5. Table 6 shows the elemental analysis values of these chromene compounds and the calculated values and characteristic ¹H-NMR spectra obtained from the structural formulas of the compounds. Table 7 shows the elemental analysis values of naphthol compounds used in Examples 2 to 16 and calculated values and characteristic ¹H-NMR spectra obtained from the structural formulas of the compounds.

**Table 1**

| Example No. | Raw materials | | Product | Yield (%) |
|---|---|---|---|---|
| | Naphthol compound | Propargyl alcohol compound | | |
| 2 | 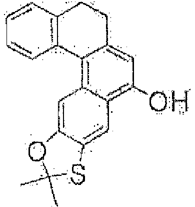 | 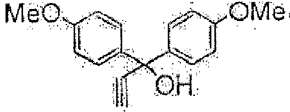 | 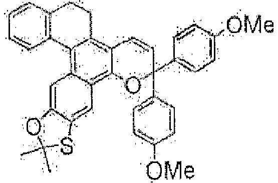 | 71 |
| 3 | 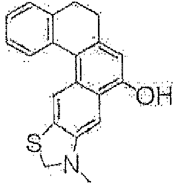 | 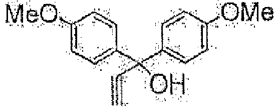 | 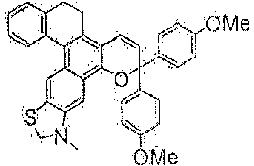 | 73 |
| 4 | 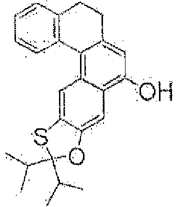 | 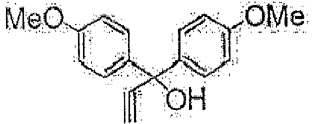 | 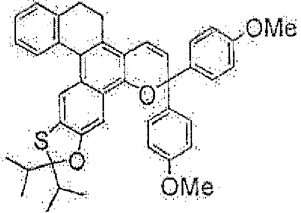 | 70 |

**Table 2**

| Example No. | Raw materials | | Product | Yield (%) |
|---|---|---|---|---|
| | Naphthol compound | Propargyl alcohol compound | | |
| 5 | 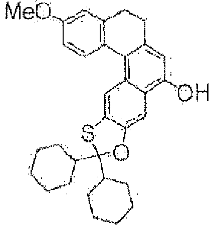 | 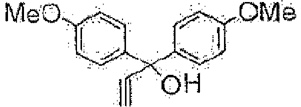 | 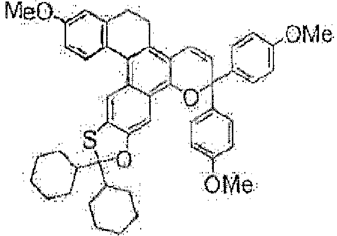 | 68 |
| 6 | 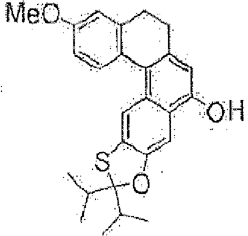 | 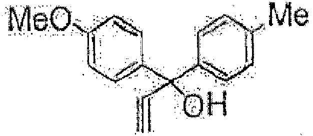 | 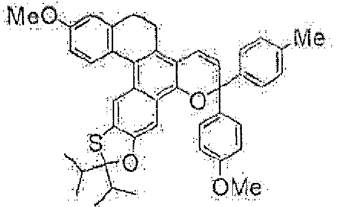 | 72 |
| 7 | 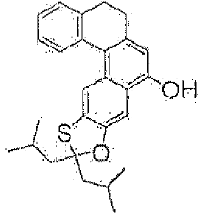 | 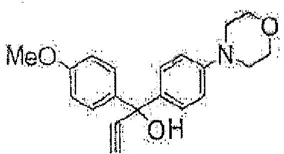 | 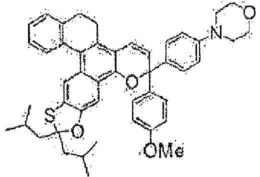 | 77 |

**Table 3**

| Example No. | Raw materials | | Product | Yield (%) |
|---|---|---|---|---|
| | Naphthol compound | Propargyl alcohol compound | | |
| 8 | 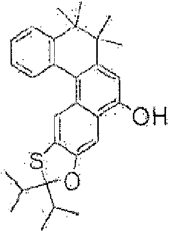 | 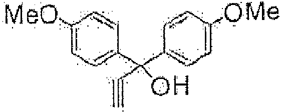 | 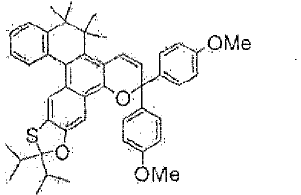 | 66 |
| 9 | 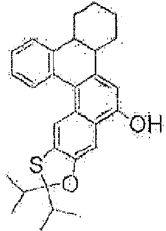 | 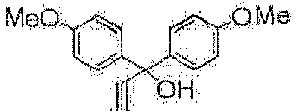 | 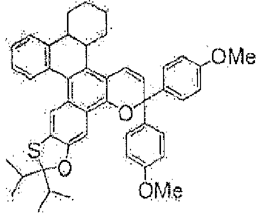 | 69 |
| 10 | 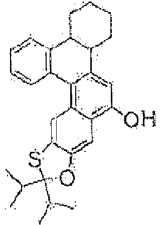 | 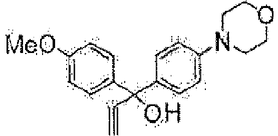 | 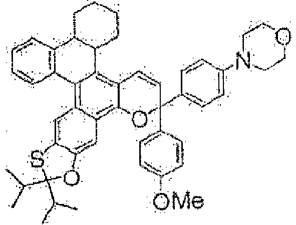 | 73 |

**Table 4**

| Example No. | Raw materials | | Product | Yield (%) |
|---|---|---|---|---|
| | Naphthol compound | Propargyl alcohol compound | | |
| 11 | 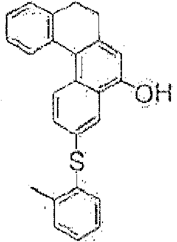 | 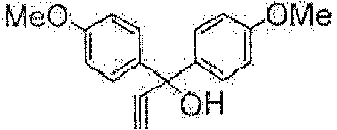 | 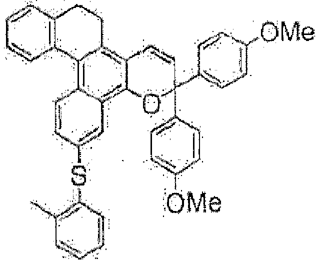 | 64 |
| 12 | 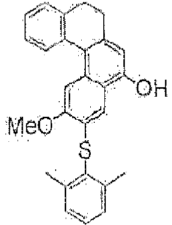 | 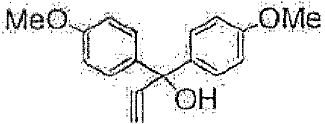 | 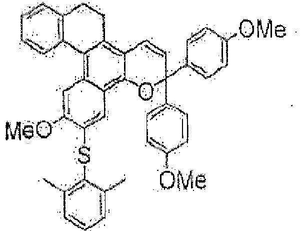 | 61 |
| 13 | 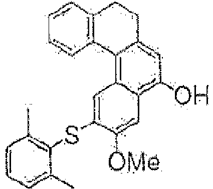 | 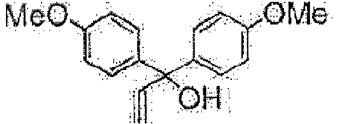 | 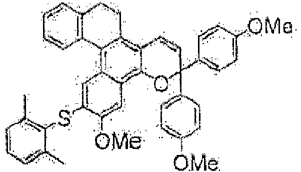 | 65 |

**Table 5**

| Example No. | Raw materials | | Product | Yield (%) |
|---|---|---|---|---|
| | Naphthol compound | Propargyl alcohol compound | | |
| 14 | 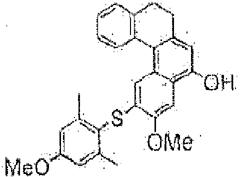 | 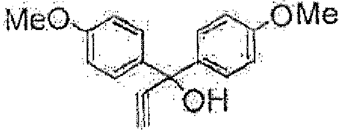 | 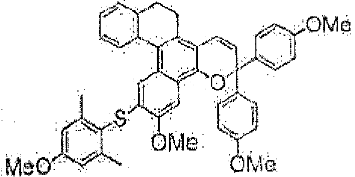 | 68 |
| 15 | 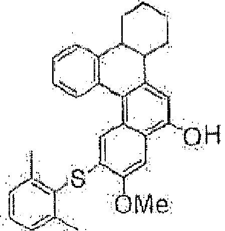 | 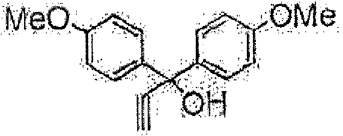 | 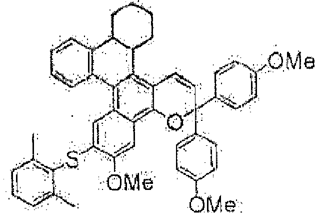 | 63 |
| 16 | 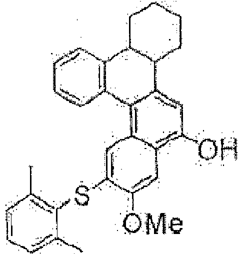 | 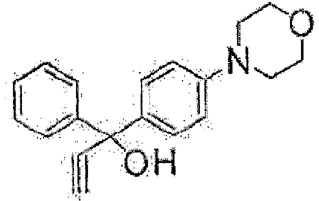 | 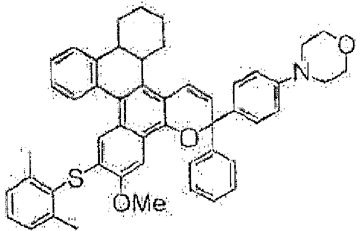 | 72 |

**Table 6**

| Ex. No. | Experimental values (chromene compound) | | | | Calculated values (chromene compound) | | | | ¹H-NMR (ppm) |
|---|---|---|---|---|---|---|---|---|---|
| | C | H | N | S | C | H | N | S | |
| 2 | 79.40 | 6.26 | | 4.77 | 79.25 | 6.35 | | 4.81 | δ 5.0-9.0 16H |
| | | | | | | | | | δ 0.5-4.9 26H |
| 3 | 78.13 | 5.50 | 2.41 | 5.68 | 78.00 | 5.48 | 2.46 | 5.63 | δ 5.0-9.0 16H |
| | | | | | | | | | δ 0.5-4.9 25H |
| 4 | 78.85 | 6.13 | | 4.94 | 78.72 | 6.29 | | 5.00 | δ 5.0-9.0 16H |
| | | | | | | | | | δ 0.5-4.9 24H |
| 5 | 78.20 | 6. 63 | | 4.31 | 78.37 | 6.71 | | 4.27 | δ 5.0-9.0 15H |
| | | | | | | | | | δ 0.5-4.9 35H |
| 6 | 78.63 | 6.49 | | 5.01 | 78.87 | 6.46 | | 4.90 | δ 5.0-9.0 15H |
| | | | | | | | | | δ 0.5-4.9 37H |
| 7 | 79.13 | 6.80 | 2.13 | 4.62 | 79.26 | 6.95 | 2.05 | 4.70 | δ 5.0-9.0 16H |
| | | | | | | | | | δ 0.5-4.9 31H |
| 8 | 79.42 | 7.09 | | 4.76 | 79.27 | 6. 94 | | 4.60 | δ 5.0-9.0 16H |
| | | | | | | | | | δ 0.5-4.9 32H |
| 9 | 79.66 | 6.58 | | 4.71 | 79.50 | 6.67 | | 4.61 | δ 5.0-9.0 16H |
| | | | | | | | | | δ 0.5-4.9 31H |
| 10 | 78.27 | 7.01 | 1.63 | 4.25 | 78.47 | 6.85 | 1.87 | 4.28 | δ 5.0-9.0 16H |
| | | | | | | | | | δ 0.5-4.9 35H |
| 11 | 81.47 | 5.56 | | 5.02 | 81.52 | 5.54 | | 5.18 | δ 5.0-9.0 21H |
| | | | | | | | | | δ 0.5-4.9 13H |
| 12 | 79.90 | 5.84 | | 4.98 | 79.73 | 5.78 | | 4.84 | δ 5.0-9.0 19H |
| | | | | | | | | | δ 0.5-4.9 19H |
| 13 | 79.72 | 5.95 | | 4.91 | 79.73 | 5.78 | | 4.84 | δ 5.0-9.0 19H |
| | | | | | | | | | δ 0.5-4.9 19H |
| 14 | 78.13 | 5.93 | | 4.64 | 78.01 | 5.82 | | 4.63 | δ 5.0-9.0 18H |
| | | | | | | | | | δ 0.5-4.9 22H |
| 15 | 80.54 | 6.14 | | 4.48 | 80.41 | 6.19 | | 4.47 | δ 5.0-9.0 19H |
| | | | | | | | | | δ 0.5-4.9 25H |
| 16 | 81.09 | 6.34 | 1.98 | 4.23 | 80.94 | 6.38 | 1.89 | 4.32 | δ 5.0-9.0 20H |
| | | | | | | | | | δ 0.5-4.9 27H |

| | | | | | | | | | |
|---|---|---|---|---|---|---|---|---|---|
| Ex.: Example | | | | | | | | | |

**Table 7**

| Ex. No. | | | | | Calculated values (chromene compound) | | | | ¹H-NMR (ppm) |
|---|---|---|---|---|---|---|---|---|---|
| | C | | N | S | C | H | N | S | |
| 2 | 75.32 | 5.57 | | 9.53 | 75.42 | 5.43 | | 9.59 | δ 5.0-9.0 8H |
| | | | | | | | | | δ 0.5-4.9 10H |
| 3 | 75.34 | 5.33 | 4.55 | 9.90 | 75.20 | 5.36 | 4.39 | 10.04 | δ 5.0-9.0 8H |
| | | | | | | | | | δ 0.5-4.9 9H |
| 4 | 76.85 | 6.66 | | 8.12 | 76.89 | 6.71 | | 8.21 | δ 5.0-9.0 8H |
| | | | | | | | | | δ 0.5-4.9 16H |
| 5 | 76.67 | 7.19 | | 6.44 | 76.76 | 7.25 | | 6.40 | δ 5.0-9.0 17H |
| | | | | | | | | | δ 0.5-4.9 16H |
| 6 | 74.44 | 6.81 | | 7.56 | 74.25 | 6.71 | | 7.62 | δ 5.0-9.0 7H |
| | | | | | | | | | δ 0.5-4.9 21H |
| 7 | 77.27 | 7.19 | | 7.83 | 77.47 | 7.22 | | 7.66 | δ 5.0-9.0 8H |
| | | | | | | | | | δ 0.5-4.9 31H |
| 8 | 77.74 | 7.49 | | 7.24 | 77.98 | 7.67 | | 7.18 | δ 5.0-9.0 16H |
| | | | | | | | | | δ 0.5-4.9 32H |
| 9 and 10 | 78.38 | 7.25 | | 7.43 | 78.34 | 7.25 | | 7.21 | δ 5.0-9.0 16H |
| | | | | | | | | | δ 0.5-4.9 31H |
| 11 | 81.47 | 5.31 | | 8.49 | 81.49 | 5.47 | | 8.70 | δ 5.0-9.0 21H |
| | | | | | | | | | δ 0.5-4.9 13H |
| 12 | 78.75 | 5.93 | | 8.00 | 78.61 | 5.86 | | 7.77 | δ 5.0-9.0 11H |
| | | | | | | | | | δ 0.5-4.9 13H |
| 13 | 78.46 | 5.65 | | 7.77 | 78.61 | 5.86 | | 7.77 | δ 5.0-9.0 11H |
| | | | | | | | | | δ 0.5-4.9 13H |
| 14 | 75.78 | 5.95 | | 7.32 | 75.99 | 5. 92 | | 7.25 | δ 5.0-9.0 108H |
| | | | | | | | | | δ 0.5-4.9 22H |
| 15 and 16 | 79.79 | 6.35 | | 6.81 | 79.79 | 6.48 | | 6.87 | δ 5.0-9.0 11H |
| | | | | | | | | | δ 0.5-4.9 19H |

| | | | | | | | | | |
|---|---|---|---|---|---|---|---|---|---|
| Ex.: Example | | | | | | | | | |

### Examples 17 to 32

### (evaluation of physical properties of photochromic plastic lenses)

0.03 parts by mass of the chromene compound obtained in Example 1, 20 parts by mass of tetraethylene glycol dimethacrylate, 50 parts by mass of 2,2-bis[4-(methacryloxyethoxy)phenyl]propane, 10 parts by mass of trimethylolpropane trimethacrylate, 9 parts by mass of glycidyl methacrylate and 1 part by mass of t-butylperoxy-2-ethyl hexanoate as a polymerization initiator were fully mixed together to prepare a photochromic curable composition. Then, the obtained composition was injected into a casting mold composed of a glass plate and a gasket made of an ethylene-vinyl acetate copolymer to carry out casting polymerization. Polymerization was carried out by using an air furnace, gradually increasing the temperature from 30 to 90°C over 18 hours and keeping the temperature at 90°C for 2 hours. After the end of polymerization, the obtained polymer was taken out from the glass casting mold to obtain a photochromic lens (thickness of 2 mm).

The following photochromic properties of the obtained photochromic plastic lens were evaluated. The results of the following evaluations using the chromene compound of Example 1 are shown in Table 8 as Example 17.
[1] Maximum absorption wavelength (λₘₐₓ) : This is the maximum absorption wavelength after color development obtained by means of the spectrophotometer (MCPD3000 instantaneous multi-channel photodetector) of Otsuka Electronics Co. , Ltd. and used as an index of color at the time of color development.
[2] Color optical density (A₀) : This is the difference between absorbance {ε(120)} after 120 seconds of exposure at the above maximum absorption wavelength and absorbance ε(0) under no exposure and used as an index of color optical density. It can be said that as this value becomes larger, photochromic properties become better.
[3] Double peak characteristic (A_{Y}/A_{B}) : This is the ratio of color optical density (A_{Y}: value of λₘₐₓ) at a yellow range (maximum absorption wavelength of 430 to 530 nm) and color optical density (A_{B}: value of λₘₐₓ) at a blue range (maximum absorption wavelength of 550 to 650 nm) and used as an index of double peak characteristic.
[4] Fading half period [τ1/2 (sec.)] : This is a time required for the reduction of the absorbance at the above maximum absorption wavelength of a sample to 1/2 of {ε(120)-ε(0)} when exposure is stopped after 120 seconds of exposure and used as an index of fading speed. As this time becomes shorter, the fading speed becomes higher.
[5] Absorption end {λ₀}: After the photochromic plastic lens obtained under the above conditions is used as a sample and kept in the dark for one day, the ultraviolet light transmittance (T%) at 300 to 800 nm of the sample is measured with an ultraviolet visible spectrophotometer (UV-2550 of Shimadzu Corporation) at room temperature. A tangent line is drawn on an ultraviolet light absorption curve obtained by plotting transmittance with respect to wavelength at a point where the transmittance (T%) on the obtained ultraviolet light absorption curve becomes 50 % so as to obtain an absorption wavelength at which the transmittance (T%) of the tangent line becomes 0 as the absorption end (absorption end of the ultraviolet light spectrum) and used as an index of initial coloration. For example, in an optical article such as a spectacle lens, as this value becomes smaller, initial coloration becomes weaker and transparency under no exposure becomes higher.
[6] Thermochromism {T₀}: The photochromic plastic lens obtained under the above conditions is used as a sample and the transmittance (T%) at 300 to 800 nm of the sample is measured with an ultraviolet visible spectrophotometer (UV-2550 of Shimadzu Corporation) at room temperature. This is a transmittance at a wavelength at which the transmittance at 430 to 650 nm becomes minimal and used as an index of initial coloration. As this value becomes larger, initial coloration becomes weaker and transparency under no exposure becomes higher.
[7] Residual rate (A₂₀₀/A₀ x 100) : A deterioration promotion test is made on the obtained photochromic plastic lens by using the X25 xenon weather meter of Suga Test Instruments Co., Ltd. for 200 hours. Thereafter, the above color optical density is evaluated before and after the test by measuring the color optical density (A₀) before the test and the color optical density (A₂₀₀) after the test in order to obtain the ratio (A₂₀₀/A₀) of these values as residual rate which is used as an index of color development durability. As the residual rate becomes higher, color development durability becomes higher.
[8] heat resistance: The change rate of the above-described double peak characteristic (A_{Y}/A_{B}) is defined as {1-( AY/AB after heating test)/(A_{Y}/A_{B} before heating test}} and the obtained value is evaluated as an index of color drift of developed color. It can be said that as the change rate of the double peak characteristic becomes lower, color drift by heating becomes smaller and heat resistance becomes higher. A heating test is carried out by using a fan oven to keep the temperature at 110°C for 12 hours.

Table 8 shows the results obtained when the above evaluations were made by carrying out the above operation using the chromene compounds produced in Examples 2 to 16 in place of the chromene compound produced in Example 1. The compound Nos. of Table 8 indicate the chromene compounds obtained in Examples 1 to 16 (for example, the chromene compound produced in Example 1 is compound No. 1 and the chromene compound produced in Example 2 is compound No. 2).

**Table 8**

| Ex. No | Co. No. | λ max | Color optical density | Double peak characteristic | Fading half period | Initial coloration (absorption end) | Initial coloration (thermochromism) | Residual rate | Double peak characteristic after heating | Color drift |
|---|---|---|---|---|---|---|---|---|---|---|
| | | (nm) | A₀ | A_{Y}/A_{B} | τ 1/2 (sec) | (nm) | (%) | (A₂₀₀ / A₀) X 100(%) | A_{Y}'/A_{B}' | 1- (A_{Y}' / A_{B}') / (A_{Y} /A_{B}) |
| 17 | 1 | 453 | 0.59 | 1.58 | 51 | 405 | 90 | 89 | 1.32 | 0.16 |
| | | 569 | 0.37 | | 51 | | 90 | 89 | | |
| 18 | 2 | 452 | 0.56 | 1.57 | 43 | 400 | 88 | 90 | 1.34 | 0.14 |
| | | 570 | 0.36 | | 43 | | 88 | 90 | | |
| 19 | 3 | 472 | 0.72 | 1.76 | 51 | 408 | 86 | 88 | 1.61 | 0.09 |
| | | 588 | 0.41 | | 51 | | 88 | 88 | | |
| 20 | 4 | 453 | 0.60 | 1.67 | 39 | 402 | 90 | 87 | 1.61 | 0.03 |
| | | 571 | 0.36 | | 39 | | 90 | 87 | | |
| 21 | 5 | 472 | 0.71 | 1.39 | 47 | 405 | 87 | 88 | 1. 67 | 0.01 |
| | | 580 | 0.42 | | 47 | | 89 | 88 | | |
| 22 | 6 | 466 | 0.74 | 1.77 | 54 | 405 | 86 | 8. | 1.76 | 0.01 |
| | | 578 | 0.42 | | 54 | | 88 | 83 | | |
| 23 | 7 | 484 | 0.65 | 1.15 | 40 | 400 | 85 | 89 | 1.09 | 0.05 |
| | | 583 | 0.56 | | 40 | | 85 | 89 | | |
| 24 | 8 | 450 | 0.66 | 1.53 | 46 | 401 | 87 | 90 | 1.50 | 0.02 |
| | | 568 | 0.43 | | 46 | | 88 | 90 | | |

| (nm) | A₀ | A_{Y}/A_{B} | τ 1/2 (sec) | (nm) | (%) | (A₂₀₀/A₀) X 100 (%) | A_{Y}' /A_{B}' | 1- (A_{Y}' / A_{B}') / (A_{Y} /A_{B}) | | |
|---|---|---|---|---|---|---|---|---|---|---|
| 25 | 9 | 448 | 0.54 | 1.50 | 33 | 400 | 90 | 92 | 1.48 | 0.01 |
| | | 566 | 0.36 | | 33 | | 91 | 92 | | |
| 26 | 10 | 468 | 0.48 | 1.21 | 32 | 400 | 91 | 92 | 1.20 | 0.01 |
| | | 582 | 0.40 | | 32 | | 91 | 92 | | |
| 27 | 11 | 460 | 0.76 | 1.26 | 49 | 410 | 87 | 88 | 1.21 | 0.04 |
| | | 575 | 0.60 | | 49 | | 88 | 88 | | |
| 28 | 12 | 451 | 0.79 | 1.61 | 58 | 399 | 85 | 86 | 1.59 | 0.01 |
| | | 568 | 0.49 | | 58 | | 87 | 86 | | |
| 29 | 13 | 450 | 0.76 | 1.66 | 54 | 404 | 86 | 88 | 1.63 | 0.02 |
| | | 571 | 0.46 | | 54 | | 88 | 88 | | |
| 30 | 14 | 453 | 0.77 | 1.68 | 57 | 400 | 84 | 86 | 1.60 | 0.05 |
| | | 572 | 0.46 | | 57 | | 88 | 86 | | |
| 31 | 15 | 448 | 0.61 | 1.50 | 38 | 403 | 86 | 90 | 1.47 | 0.02 |
| | | 564 | 0.41 | | 38 | | 88 | 90 | | |
| 32 | 16 | 470 | 0.58 | 1.33 | 36 | 403 | 87 | 91 | 1.30 | 0.02 |
| | | 572 | 0.43 | | 36 | | 88 | 91 | | |

| | | | | | | | | | | |
|---|---|---|---|---|---|---|---|---|---|---|
| Ex.: Example, Co.: Compound | | | | | | | | | | |

### Examples 33 to 36

Various naphthol compounds were synthesized in the same manner as in Example 1 to synthesize chromene compounds shown in Table 9 therefrom. When the structures of the obtained products were analyzed by using the same structure confirming means as in Example 1, it was confirmed that they were compounds represented by structural formulas shown in Table 9. Table 10 shows the elemental analysis values of these chromene compounds and the calculated values and characteristic ¹H-NMR spectra obtained from the structural formulas of the compounds. Table 11 shows the elemental analysis values of the naphthol compounds used in Examples 33 to 36 and the calculated values and characteristic ¹H-NMR spectra obtained from the structural formulas of the compounds.

**Table 9**

| Ex. No. | Raw materials | | Product | Yield (%) |
|---|---|---|---|---|
| | Naphthol compound | Propargyl alcohol compound | | |
| 33 | 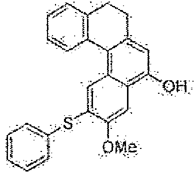 | 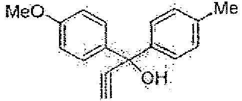 | 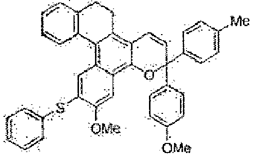 | 61 |
| 34 | 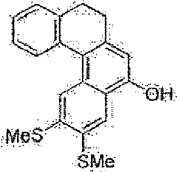 | 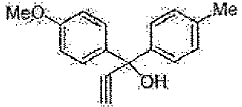 | 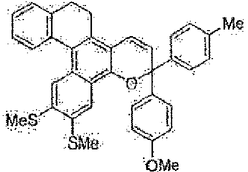 | 56 |
| 35 | 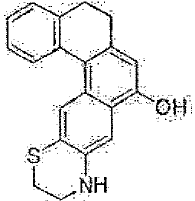 | 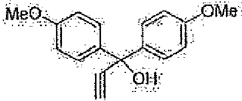 | 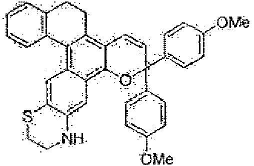 | 49 |
| 36 | 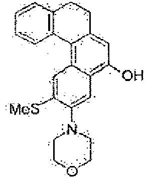 | 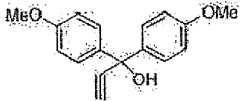 | 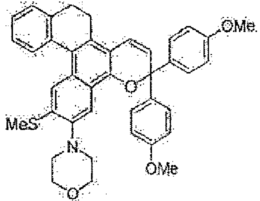 | 66 |

| | | | | |
|---|---|---|---|---|
| Ex.: Example | | | | |

**Table 10**

| Ex. No. | Experimental values (chromene compound) | | | | Calculated values (chromene compound) | | | | ¹H-NMR (ppm) |
|---|---|---|---|---|---|---|---|---|---|
| | C | H | N | S | C | H | N | S | |
| 33 | 81. 60 | 5.57 | | 5.29 | 81.52 | 5.54 | | 5.18 | δ 5.0-9.0 21H |
| | | | | | | | | | δ 0.5-4.9 13H |
| 34 | 77.47 | 5.86 | | 11.16 | 77.59 | 5.63 | | 11.20 | δ 5.0-9.0 16H |
| | | | | | | | | | δ 0.5-4.9 16H |
| 35 | 77.80 | 5.55 | 2.36 | 5.76 | 78.00 | 5.48 | 2.46 | 5.63 | δ 5.0-9.0 16H |
| | | | | | | | | | δ 0.5-4.9 15H |
| 36 | 76.56 | 6.00 | 2.39 | 5.01 | 76.53 | 5.94 | 2.23 | 5.11 | δ 5.0-9.0 13H |
| | | | | | | | | | δ 0.5-4.9 21H |

| | | | | | | | | | |
|---|---|---|---|---|---|---|---|---|---|
| Ex.: Example | | | | | | | | | |

**Table 11**

| Ex. No. | Experimental values (naphthol compound) | | | | Calculated values (naphthol compound) | | | | ¹H-NMR (ppm) |
|---|---|---|---|---|---|---|---|---|---|
| | C | H | N | S | C | H | N | S | |
| 33 | 77.94 | 5.14 | | 8.52 | 78.09 | 5.24 | | 8.34 | δ 5.0-9.0 12H |
| | | | | | | | | | δ 0.5-4.9 8H |
| 34 | 70.79 | 5.33 | | 19.14 | 70.97 | 5.36 | | 18.95 | δ 5.0-9.0 7H |
| | | | | | | | | | δ 0.5-4.9 11H |
| 35 | 75.31 | 5.22 | 4.42 | 10.14 | 75.20 | 5.36 | 4.39 | 10.04 | δ 5.0-9.0 7H |
| | | | | | | | | | δ 0.5-4.9 10H |
| 36 | 73.04 | 6.24 | 3.72 | 8.55 | 73.18 | 6.14 | 3.71 | 8.49 | δ 5.0-9.0 7H |
| | | | | | | | | | δ 0.5-4.9 16H |

| | | | | | | | | | |
|---|---|---|---|---|---|---|---|---|---|
| Ex.: Example | | | | | | | | | |

### Examples 37 to 40

### (evaluation of physical properties of photochromic plastic lenses)

Photochromic plastic lenses were obtained from the chromene compounds produced in Examples 33 to 36 to evaluate their characteristic properties. The results are shown in Table 12.

**Table 12**

| Ex. No. | Co. No. | λ max | Color optical density | Double peak characteristic | Fading half period | Initial coloration (absorption end) | Initial coloration (thermochromism) | Residual rate | Double peak characteristic after heating | Color drift |
|---|---|---|---|---|---|---|---|---|---|---|
| | | (nm) | A₀ | A_{Y}/A_{B} | τ 1/2 (sec) | (nm) | (%) | (A₂₀₀/A₀) X 100 (%) | A_{Y}'/A_{B}' | 1- (A_{Y}' /A_{B}') / (A_{Y}/A_{B}) |
| 37 | 33 | 448 | 0.79 | 1.68 | 60 | 408 | 85 | 87 | 1.39 | 0.17 |
| | | 567 | 0.47 | | 60 | | 88 | 87 | | |
| 38 | 34 | 452 | 0.61 | 1.64 | 52 | 408 | 90 | 86 | 1.35 | 0.18 |
| | | 570 | 0.37 | | 52 | | 90 | 86 | | |
| 39 | 35 | 472 | 0.7 | 1.37 | 66 | 411 | 84 | 88 | 1.51 | 0.09 |
| | | 588 | 0.42 | | 66 | | 85 | 88 | | |
| 40 | 36 | 455 | 0.66 | 1.61 | 64 | 414 | 83 | 90 | 1.38 | 0.14 |
| | | 573 | 0.41 | | 64 | | 83 | 90 | | |

| | | | | | | | | | | |
|---|---|---|---|---|---|---|---|---|---|---|
| Ex.: Example Co.: Compound | | | | | | | | | | |

### Comparative Examples 1 to 3

For comparison, photochromic plastic lenses were obtained from compounds represented by the following formulas (A), (B) and (C) in the same manner as in Examples to evaluate their characteristic properties. The results are shown in Table 13.

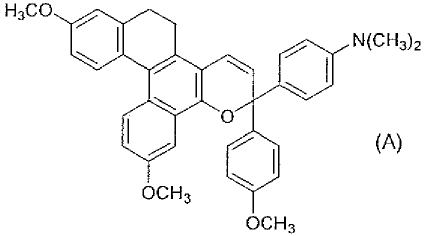

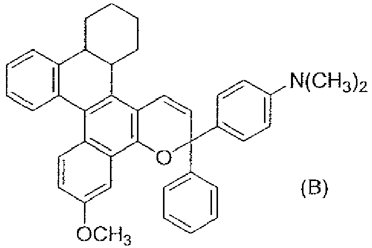

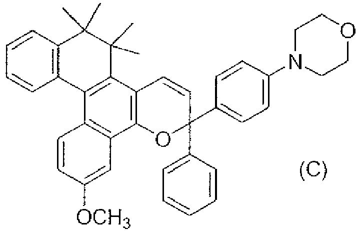

**Table 13**

| Co. Ex. No. | Compound No. | λ max | Color optical density | Double peak characteristic | Fading half period | Initial coloration (absorption end) | Initial coloration (thermo-chromism) | Residual rate |
|---|---|---|---|---|---|---|---|---|
| | | (nm) | A₀ | A_{Y}/A_{B} | τ 1/2 (sec) | (nm) | (%) | (A₂₀₀/A₀) X 100 (%) |
| 1 | (A) | 496 | 0.83 | 1.09 | 285 | 404 | 78 | 71 |
| | | 594 | 0.76 | | 285 | | 79 | 71 |
| 2 | (B) | 498 | 0.85 | 1.12 | 78 | 403 | 75 | 80 |
| | | 590 | 0.76 | | 78 | | 76 | 80 |
| 3 | (C) | 482 | 0.73 | 0.92 | 92 | 82 | 86 | 86 |
| | | 592 | 0.79 | | 92 | | 81 | 86 |

| | | | | | | | | |
|---|---|---|---|---|---|---|---|---|
| Co.Ex.: Comparative Example | | | | | | | | |

It is understood that the photochromic plastic lenses of Examples 17 to 32 and Examples 37 to 40 which were obtained from the chromene compounds of the present invention have excellent performance such as high double peak characteristic, high fading speed, little initial coloration by thermochromism and high repeat durability as compared with the,photochromic plastic lenses of Comparative Example 1 (chromene compound represented by the above formula (A)), Comparative Example 2 (chromene compound represented by the above formula (B)) and Comparative Example 3 (chromene compound represented by the above formula (C)).

### Effect of the Invention

The chromene compound of the present invention develops a color of a neutral tint and has little initial coloration, high color development sensitivity, high color optical density, high fading speed even when it is dispersed in a solution or a polymer solid matrix and excellent durability.

Therefore, for example, a photochromic lens can be manufactured by using the chromene compound of the present invention, which develops a color of a strong neutral tint swiftly when it goes outside and fades swiftly to return to its original color when it returns inside from outside and has such high durability that it can be used for a long time.

## Claims

1. A chromene compound represented by the following formula (1). 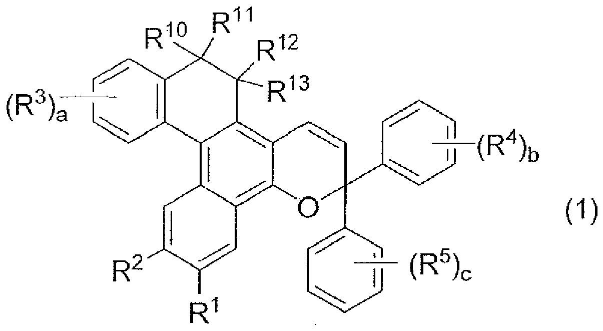 {In the above formula, R¹ and R² are defined by the following (i) or (ii) :
(i) Either one or both of R¹ and R² are sulfur-containing groups represented by the following formula (2), or either one of them is a sulfur-containing group represented by the formula (2) and the other is a hydrogen atom, hydroxyl group, alkyl group, haloalkyl group, cycloalkyl group, alkoxy group, amino group, heterocyclic group which may have a substituent and bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group.
-S-R⁶ (2)
(wherein R⁶ is a hydrogen atom, alkyl group, cycloalkyl group or aryl group.)
(ii) R¹ and R² are bonded together to form a group represented by the following formula (3) : 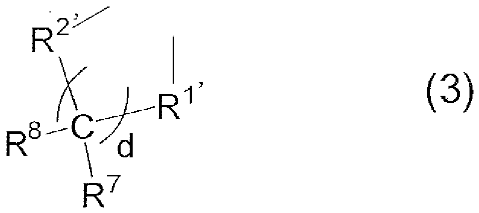 (wherein either one or both of R¹' and R²' are sulfur-atoms, or either one of them is a sulfur atom and the other is a methylene group, oxygen atom or group represented by the following formula (4): 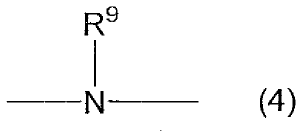 (wherein R⁹ is a hydrogen atom, hydroxyl group, alkyl group, haloalkyl group, cycloalkyl group, alkoxy group, halogen atom, aralkyl group, aralkoxy group, aryloxy group or aryl group.)
R⁷ and R⁸ are each a hydrogen atom, hydroxyl group, alkyl group, haloalkyl group, cycloalkyl group, alkoxy group, amino group, heterocyclic group bonded to a carbon atom bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group or aryl group, R⁷ and R⁸ may be bonded together to form an aliphatic ring having 3 to 20 ring member carbon atoms together with carbon atoms bonded thereto, "d" is an integer of 1 to 3.)
R³, R⁴ and R⁵ are each a hydroxyl group, alkyl group, haloalkyl group, cycloalkyl group, alkoxy group, amino group, heterocyclic group bonded to a benzene ring bonded thereto via a ring member nitrogen atom, cyano group, nitro group, formyl group, hydroxycarbonyl group, alkylcarbonyl group, alkoxycarbonyl group, halogen atom, aralkyl group, aralkoxy group, aryloxy group, aryl group, alkylthio group, cycloalkylthio group or arylthio group, "a" is an integer of 0 to 4, "b" and "c" are each an integer of 0 to 5, when "a" is 2 to 4, a plurality of R³' s may be the same or different, and when "b" and "c" are each 2 to 5, a plurality of R⁴'s and a plurality of R⁵'s may be the same or different, R¹⁰, R¹¹, R¹² and R¹³ are each a hydrogen atom, alkyl group, cycloalkyl group or aryl group, and two groups selected from R¹⁰, R¹¹, R¹² and R¹³ may be bonded together to form a ring together with carbon atoms bonded thereto.}

2. A photochromic curable composition which comprises the chromene compound of claim 1 and polymerizable monomers.

3. A photochromic optical article having a polymer molded body containing the above chromene compound of claim 1 dispersed therein as a constituent member.

4. An optical article having an optical substrate at least one surface or all of which is covered with a polymer film containing the chromene compound of claim 1 dispersed therein as a constituent member.

5. A naphthol compound represented by the following formula (5). 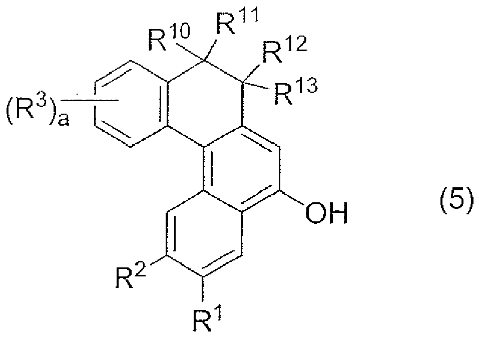 (wherein R¹, R², R³, R¹⁰, R¹¹, R¹², R¹³ and "a" are as defined in the above formula (1).)
